# EUROPEAN PATENT APPLICATION

(11) **EP 3 135 107 A2**
(43) Date of publication of application: **01.03.2017**
(21) Application number: 16202140.6
(22) Date of filing: 05.12.2016
(51) Int. Cl.: A01K 67/033, A61K 8/98, A61L 2/00, A61L 2/18

(54) **USE OF AN OZONIZATION DEVICE FOR THE EXTRACTION OF SNAIL SLIME AND RELATED DEVICE, STIMULATING SOLUTION AND PROCESS FOR THE EXTRACTION OF SNAIL SLIME**

(30) Priority: 07.12.2015 IT UB20156858
(71) Applicant: Donatella Veroni GmbH, 9500 Villach Kärnten (AT)
(72) Inventor: Veroni, Donatella, 9500 Villach (AT); Franzoni, Gian Maria, 46031 Bagnolo San Vito (MN) (IT)
(74) Representative: Münchow, Vera Ute Barbara

(57) **Abstract**

The invention concerns the use of a device (10) comprising (a) at least one container (14, 16) with at least one hole (38, 28) and its lid (20, 22) to close the container (14, 16) hermetically, where the container (14, 16) or the lid (20, 22) is provided with at least an inlet (18, 24, 26) in order to introduce a stimulating solution or sterilized and/or ozonated water; (b) a sanitizing unit comprising an ozone generator (32) and a sterilization device (36), and which can be supplied or is supplied by a water supply; (c) one or more tanks (40) for containing or that contain a stimulating solution; and (d) a liquid transport system, for the transfer of the stimulating solution and the sterilized and/or ozonated water respectively from said one or more tanks (40) and from said sanitizing unit through said inlet (18, 24, 26) to said at least one container (14, 16); wherein said at least one inlet (18, 24, 26) is connectable or is connected with said one or more tanks (40) or said sanitizing unit in order to be supplied with said stimulating solution or said sterilized and/or ozonated water for the extraction of snail slime. The invention further concerns a relative device, a stimulation solution and a process for extracting snail slime.

## Description

### TECHNICAL FIELD

The present invention relates to a use of an ozonization device for the extraction of snail slime, a corresponding device and a process for the extraction of snail slime, and a respective stimulating solution to stimulate the production of slime by the snail. The snail slime finds application in, for example, cosmetics, drugs, supplements and veterinary products.

### BACKGROUND ART

The snail is an invertebrate animal belonging to the *phylum* of molluscs. Its slow movement is well known; in fact, it drags itself by means of the foot and uses a slime as a lubricant to avoid injury. The secretions of the snail also serve to form the epiphragm when the animal withdraws into its shell. In addition, the snail uses the slime for protection from drying and to adhere to surfaces difficult to access.

For cosmetics, a special interest in the use of snail slime is emerged, in the form of a concentrate of nutritive and regenerative substances derived primarily from a common invertebrate in our gardens, the snail *Helix aspersa (Müller).* This species produces a mucous secretion consisting of several substances, some of which are of great interest in cosmetics, such as allantoin (a substance that stimulates the regeneration of tissues), glycolic acid (that allows to exfoliate the superficial layers of the skin and stimulates directly the formation of collagen in the dermis), elastin, collagen (a moisturizing and softening agent) and certain vitamins and proteins, and mucopolysaccharides.

As a cosmetic ingredient, the slime is recommended for an acne-prone skin, to speed the repair of the epidermis and reduce the formation of acne marks, to ameliorate the discolouration and to improve the aesthetic visibility of scars, but also to prevent the stretch marks and as anti-ageing and epithelial renewing agent, even in cases of dermatitis, redness and irritation. Usually, the slime extraction is performed in conditions in which the animal is stressed, and the results are not satisfactory from the point of view of the composition of the slime. These extraction methods comprise the application of water and salt, ionizing radiation, electric shocks, vibrations or centrifugations on the snail. These methods often result in high mortality of the animals, which can also exceed 80%.

### DISCLOSURE OF THE INVENTION

Object of the invention is to propose a process for the extraction of snail slime that is fast and easy to be executed, and a corresponding device and auxiliary means in order to perform the extraction procedure, in which the snail is not stressed, and in which the slime obtained is without pathogenic germ contaminations and includes significant concentrations of substances useful for the cosmetic treatment of skin.

The object is achieved by a use of a device which comprises at least one container with at least one hole and a respective lid to close the container hermetically, where the container or the lid is provided with at least an inlet to introduce a stimulating solution or sterilized and/or ozonated water; a sanitizing unit comprising an ozone generator and a sterilization device, preferably a UV lamp or a reverse osmosis device, which can be supplied or is supplied by a water supply; one or more tanks for containing or that contain a stimulating solution; and a liquid transport system, for the transfer of the stimulating solution or sterilized and/or ozonated water, respectively, from said one or more tanks or from said sanitizing unit, through said inlet, to said at least one container; wherein said at least one inlet is connectable or connected (for example through a pipe) with said one or more tanks or said sanitizing unit in order to be supplied by said stimulating solution or sterilized and/or ozonated water for the extraction of the snail slime.

The use of ozonization devices for the extraction of snail slime is not known in the art, but it is just the ozonization step that makes the extraction according to the invention particularly mild and not very harmful to the snail.

The hermetic closure of the containers allows a treatment of snails without contact with the outside, thus avoiding the introduction of bacteria, dirt, etc.

The hole of the container allows to drain the sterilized and/or ozonated water used for washing the snails or the collection of the slime. The hole can be provided with a tap or valve to allow the regulated closing and opening thereof.

Another aspect of the invention provides a device for the extraction of the snail slime which includes at least a first and a second container, each with at least one hole and a corresponding lid to close the container hermetically, where each container or its lid is provided with at least one inlet to introduce a stimulating solution or sterilized and/or ozonated water; a sanitizing unit comprising an ozone generator and a sterilization device, preferably a UV lamp or a reverse osmosis device, which can be supplied or is supplied by a water supply; one or more tanks for containing or that contain a stimulating solution; and a liquid transport system, for the transfer of the stimulating solution from said one or more tanks, through the respective inlet, to said second container, and for the transfer of the sterilized and/or ozonated water from said sanitizing unit, through the respective inlet, to said first or second container; wherein said at least one inlet of the first container is connectable or is connected (for example through a pipe) with said sanitizing unit in order to be supplied with sterilized and/or ozonated water, and wherein said at least one inlet of the second container is connectable or is connected with said one or more tanks or said sanitizing unit in order to be supplied with said stimulating solution or sterilized and/or ozonated water. This device is particularly suitable for use according to the invention or for carrying out the process according to the invention, which will be described later. The device allows to achieve a treatment in two steps, namely an ozonization step and a stimulation step. State-of-the-art ozonization washing devices, as for example US 2010/122713 A1 (washing of fruits and vegetables), US 6,379,617 B1 (disinfection of a dialysis machine), US 2015/306266 A1 (cleaning of articles from spores), JP 2001/149470 A (sanitation of waste water from a dialysis machine) and US 2007/251549 A1 (dishwasher), do not provide the possibility to treat introduced articles (or animals) with two different solutions in two separate containers. None of the above documents provides for a configuration with two containers and their particular connection to specific supplies of each container, i.e., to a supply of ozonated water and a stimulating solution.

This variant allows separating the washing step from the stimulation step of the snails to speed the extraction process without having to change the inlet connections to the supply of ozonated water or to the supply of stimulating solution, a change that would be otherwise necessary if there was only a single container.

In a very preferred embodiment of the invention, in the device for the extraction of the snail slime according to the invention the second container or its lid comprises a first and a second inlet, where the first inlet is connectable or is connected (for example through a pipe) with the sanitizing unit, and where the second inlet is connectable or is connected with said one or more tanks to contain or containing the stimulating solution.

Both the inlets may be located in the second container or both in the lid, and analogously an inlet can be located in the container and the other in the lid.

Obviously, it is also conceivable that a single container system with a lid that includes two inlets to realize two distinct stable connections for the sterilized and/or ozonated water and the stimulating solution. Both the inlets can be located either in the container or in the lid, or the one can be arranged in the container and the other in the lid.

The presence of two inlets avoids having to change the supply between the individual steps forming part of the stimulation of the animals, and having to clean the inlet at every supply change, if needed.

Embodiments of the invention may provide devices with more than two containers to perform the washing and extraction steps in several containers simultaneously. The presence of the ozone generator allows the ozone production and thus the ozonated water production when needed (just-in-time), thus avoiding the decomposition of ozone during the possible storage in tanks.

Advantageously, the inlet(s) of the container(s) is/are equipped with a spray device. To perform the extraction process according to the invention, which will be explained later, it is useful to introduce the sterilized and/or ozonated water as well as the stimulating solution as a spray in the closed treatment container to allow a more even treatment of the snails and to maintain them in an environment saturated with sterilized and/or ozonated water or stimulating solution. At the same time, it keeps intact the well-being of the mollusc to avoid a drowning in an excess of water flow. The nebulization is designed specifically to prevent damage to the mollusc.

In a preferred embodiment, the device has a heating means for the water and/or the stimulating solution, and/or a heating means for the container(s). Preferably, the animals are treated at temperatures near 20°C, advantageously at about 18°C.

After extensive research, the inventors have found an alternative to water and salt treatments, ionizing rays, centrifugation, electric shocks etc. to stimulate the glands of snails to produce their secretions, i.e. the slime. For such stimulation, particularly if preceded by an ozonization treatment, the application of an acid stimulating solution proved to be very useful and mild for the animal itself.

An aspect of the invention relates to a solution for the stimulation of production of snail slime that has a pH between 1.6 and 2.4, and which comprises, as an aqueous solution, citric acid, sodium benzoate and potassium sorbate and, optionally, betaine, N,N,N-trimethylglycine (also known as N,N,N-trimethylammonium acetate) and xylitol.

Advantageously, the solutions comprise from 4 to 15% by weight, preferably from 4 to 8% by weight, more preferably about 5% by weight of citric acid, from 0.05 to 0.9% by weight, preferably from 0.05 to 0.5% by weight, more preferably 0.1 % by weight of sodium benzoate, and from 0.03 to 0.9% by weight, preferably from 0.03 to 0, 5% by weight, more preferably about 0.05% by weight of potassium sorbate. The amount of betaine and xylitol, if present, each advantageously vary between 0.5 and 2% by weight and preferably are equal to about 1 % by weight.

To the knowledge of the state of the art of the inventors, the present "chemical" stimulation of snails with glands stimulation by the above solution is not known in the art. In fact, another aspect of the invention relates to the use of the solution according to the invention for the stimulation of production of the snail slime for the extraction of the snail slime.

A further aspect of the invention relates to a process for the extraction of snail slime, which comprises the following steps:
(a) the treatment of snails, preferably of the species *Helix aspersus Müller,* with ozonated water; and
(b) the treatment of snails from step (a) with a stimulating solution according to the invention.

The treatment with ozonated water is used for the sanitation and revitalization (awakening) of the snail. Preferably, the step (a) is performed with sterilized and ozonated water. The sterilization maybe carried out using different methods, and preferred methods are the treatment with UV rays or reverse osmosis.

In step (a) the snails are cleaned and wake up, and in step (b) they are stimulated to produce their slime. This treatment is carried out in full respect of the animal and does not involve any stress to the same. The specific combination of washing with ozone and extraction with specific acid stimulating solutions has allowed to obtain an extract that was pure and rich in substances useful in cosmetic treatments.

Therefore, the extraction of the snail slime according to the invention mainly comprises two steps. The firs step is the sanitation of snails. Advantageously, the snails are subjected to a gentle washing with sterilized water by ultraviolet treatments or by reverse osmosis (hyperfiltration) and mixed with ozone. During this procedure, for a duration of preferably a few minutes, a process of revitalization and awakening of the animal also occurs. In second step the extraction of the slime itself is performed. The snails are stimulated via a nebulization with a stimulating solution composed of natural substances. The process according to the invention guarantees a non-invasive physiological stimulation, without prejudicing the health of the animal, using the same in the production cycle, thus obtaining a snail secretion that is qualitatively stable and with a significant reduction of pathogens and moulds.

Advantageously, in the process according to the invention in step (b) alternate treatments with stimulating solution according to the invention and standby steps without treatment of the snails are used. These standby steps reduce the stress on the animal.

The extract obtained with the method according to the invention is advantageously purified by microfiltration and/or the addition of a stabilizer.

In a preferred embodiment, the steps (a) and (b) are performed in a device according to the invention. Very advantageously, the device according to the invention for this purpose also comprises a basket, for example a tank provided with a sieve bottom (for the discharge of the washing solution or the collection of the slime), which can be transferred from the first to the second container to facilitate the transport of the snails from a work step to another.

The process according to the invention has enabled the production of a snail slime that appears to be totally without *Escherichia coli, Candida albicans, Staphylococcus aureus, Pseudomonas aeruginosa.* In addition, the slime appears richer in nutrients for the skin, such as allantoins, as compared to slimes obtained with the more cruel methods for the snails.

The process according to the invention has been studied to obtain a snail secretion less contaminated as possible and that does not have the need for complex processing for the storage and the use of additives or preservatives that are chemically too aggressive for both the animal and the humans, and that retains all its beneficial properties without alterations and to respect the life of the snail.

Advantageously, at the end of step (b) the snails are washed with sterilized and also, preferably, ozonated water.

The features described for one aspect of the invention may be transferred *mutatis mutandis* to the other aspects of the invention.

Said objects and advantages will be better highlighted during the description of preferred embodiment examples of the invention given, by way of example and not of limitation.

Embodiments of the invention are the object of the dependent claims. The description of preferred embodiments of the use of the extraction device, the device, the stimulating solution, its use for the extraction of the slime and the extraction process according to the invention is given by way of non-limiting example only.

### BRIEF DESCRIPTION OF THE DRAWING

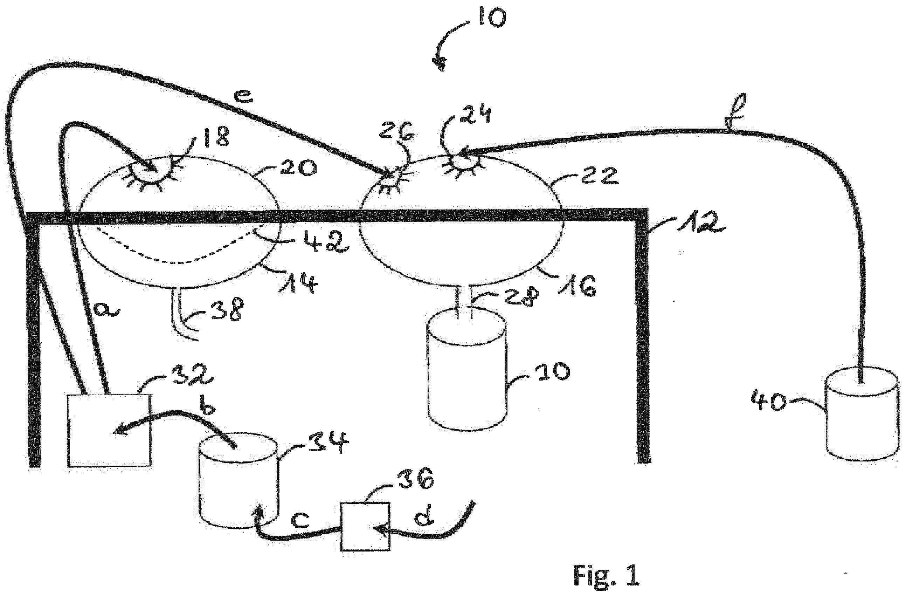

FIG. 1 illustrates in schematic form an embodiment example of a device for the extraction of the snail slime according to the invention.

### DESCRIPTION OF PREFERRED EMBODIMENT EXAMPLES

In Figure 1 a device **10** for the extraction of snail slime according to the invention is shown. The device **10** is composed of a work bench **12** where two containers **14** and **16** are housed. The first container **14** is designed for washing the snails through the spray nozzles **18** arranged in the lid **20** that closes the container **14.** The second container **16** with its lid **22** and two spray nozzles **24** and **26** is designed for the alternation between the stimulation and the rinsing steps of the snails washed previously in the first container **14** and for the collection of the snail secretion filtered through a drain **28** provided with a filter (not shown) into the vessel **30.** The first container **14** is supplied (arrow **a**) with ozonated water produced by an ozone generator **32** in turn supplied with sterilized water (arrow **b**) coming from a tank **34.** The tank **34** is in turn supplied (arrow **c**) by a flow of sterilized drinking water. The sterilized water is produced during the passage of drinking water (arrow **d**) under a UV (ultraviolet) lamp **36.** The first container **14** is provided with a drain **38** to drain the ozonated water used for washing. The two nozzles **24** and **26** are supplied with sterilized and ozonated water, respectively (alternatively, it is possible, for example, for a final wash also a supply with sterilized water only) from the UV lamp/ozone generator system (**32**, **36**) according to the arrow **e** and with a stimulating solution (arrow **f**) that is located in a tank **40.** The sterilized/ozonated water and the stimulating solution are transported be means of pump systems (not shown). In the first container **14,** a basket **42** is accommodated having a perforated bottom for containing the snails and that can be transferred (with the snails) in the container **16.**

The device **10** therefore consists substantially of two units. The first unit comprises the assembly of the UV lamp (or alternatively a reverse osmosis device) **36,** the ozone generator **32,** the tanks **34** for the collection of washing water **34** and the stimulating solution **40,** pumps and pipes (connection fittings). The second unit includes the wash snail and extraction slime work bench consisting of an operating table **12,** for example of steel or the like, with two separate containers **14** and **16,** the first container **14** for washing and sanitizing the snails, the second container **16** for the stimulation by the stimulating solution and for the collection of the snail slime.

The first container **14** is closable by an airtight plexiglass cap **20** to prevent external contaminations and includes a perforated basket **42** of steel or the like for the collection of the snails during the washing that occurs, for example, via a coil with nozzles **18** for washing. A tank (not shown) made of steel or the like, attached to the working plane, is designed for the collection of the washing water after its use. The second container **16** is closable by means of an airtight plexiglass cap **22** to prevent external contamination, which is provided with, for example, a double coil with spray nozzles **24** and **26.**

The device **10** is used in the following manner: the snails are placed in the basket **42** within the first container **14.** The sterilized and ozonated water goes through the nozzle **18** in a spray form from the UV lamp/ozone generator system (**32, 36**) controlled by timers and solenoid valves (not shown) in the container **14** for washing the snails.

Following the washing, the water is discharged through the drain **38.** Thereafter, the stimulation and rinsing operation in the second container **16** is performed. Again, the washed snails are transferred in the basket **42** within the second container **16.** The extraction procedure comprises the step of arranging the snail in a sauna at 18°C. For this purpose, the device is provided with a heating means for the water and the stimulating solution and/or with a heating means for the container. Then the stimulation is carried out by spraying the stimulating solution above the snails. Next, the snails, that were stimulated for secreting slime, are rinsed with sterilized and ozonated water through the nozzle **26.** The slime comes out from the drain **28** as a admixture with the liquids used during the extraction step.

With this extraction method, the snails are not subjected to any stressing treatments, and survival rates of 98% are regularly attained. Preferably, the filtered slime liquid collected in the vessel **30** will be subsequently further treated to ensure the absence of pathogenic bacteria and the stability of the slime.

The present device **10** is an innovative technology for extracting snail slime without stressing the animal. It consists of a complex assembly of tools already used also in the pharmaceutical industry. The device is easy to use and maintain, and it is suitable for all snail culture facilities. Thanks to airtight closed-loop processes (the lids **20** e **22** close the containers **14** e **16** hermetically), the extraction can be defined as being protected from external environments.

The extraction steps comprises the use of natural substances that do not affect the health of the snail and that actually revitalize its physiological well-being.

When the machine is switched on, the supply water is sterilized in the device **36** and deposited into the collection tank **34.** The sterilized water is ozonated by an ozone generator **32** for a greater purity and sanitization. After having previously rinsed the snails to be treated, the snail culture operator will locate the snails within the perforated steel basket **42** positioning them as a double-layer (about 700 animals) in said first "washing" container **14.**

Once ozonated, the water is transferred by a dedicated distribution system in the first container **14** for the washing, sanitizing and awakening process. This process will be carried out by using a spray for the elimination of residues and pathogens. During this step the snail is disinfected by killing any pathogens, while preserving the conditions of life of the snail. The duration of the procedure is, for example, about 15 minutes.

When finished, the plexiglass cap **20** of the first container is opened, the basket **42** containing the sanitized snails is transferred into the second container **16** by closing the second cap **22** where the process called "extraction" will be performed.

The snail slime extraction process begins with the alternation of the spraying of the stimulating solution and standby steps. The slime thus produced will be filtered and microfiltered to purify it further and collected in a storage container. Before the actual storage, the slime collected in this process we will be controlled by the person responsible for the extraction, which will be responsible for measuring acidity and pH with an appropriate tool and for adding to the extract a stabilizer to maintain and preserve the same. The extract so treated will be stored in a suitable cold room until its use. The addition of the stabilizer (preservative) takes place after the microfiltration.

After the extraction procedure, the snails will be introduced again in their culture environment where they will resume their natural biological life without stress or alterations. For respect for the animal, the snail will not be treated again for at least 40 days.

The ozone concentrations within ozonated water are approximately 300-500 ppm per litre of used water. The amount of sterilized and/or ozonated water and/or of the stimulating solution introduced in the respective containers **14** e **16** correspond to flow rates of 3 litres/hour for the sterilized and/or ozonated water and of 2 litres/hour for the stimulating solution, respectively.

Each collection litre of the secretion is stored in a cold room at a temperature of about 4°C awaiting to be microfiltered and stabilized.

The extracted snail slime is processed through a microfiltration apparatus in two steps: the first uses a 1 micron filter and then the slime is micro-filtered through a 0.45 micron filter for bacterial killing. Accessories that can be used in this process are a transparent filter-holder cup 10" ATLAS, a 1-micron sediment cartridge and a 0.45-micron sediment cartridge, and a diaphragm pump.

Following the filtrations, in order to stabilize the microfiltered slime, about 10 ml of a preservative are added for each litre of microfiltered secretion, such as for example a mixture composed of 40% by weight of water, 40% by weight of benzyl alcohol, 15% by weight of sodium benzoate, and 5% by weight of potassium sorbate.

Below we propose different timing for the extraction of about 1 litre of slime:

### Example 1 :

- First rinsing to eliminate the coarse impurities (earth, little stones and grass).
- Transferring 10 kg of snails (700/720 snails) in the first container for washing with water and ozone.
- In the first container, washing for a duration of 30 min with sterilized and ozonated water.
- Transferring the washed snails in the basket within the second container for the extraction.
- Extraction: 30 sec stimulation - 8 min wait - 8 sec stimulation - 8 min wait - 8 sec stimulation - 8 minutes wait - 8 sec stimulation - 2 minutes wait - 50 seconds of final rinse with sterilized and ozonated water.
- Removing the slime collection bottle, performing a washing of 4 min of the animal with ozonated and sterilized water before going back in the culture environments.

### Example 2:

- First rinsing to eliminate the coarse impurities (earth, little stones and grass).
- Transferring 10 kg of snails (700/720 snails) in the first container for washing with water and ozone.
- In the first container, washing for a duration of 30 min with sterilized and ozonated water.
- Transferring the washed snails in the basket within the second container for the extraction.
- Extraction: 30 sec stimulation - 9 min wait - 10 sec stimulation - 9 min wait - 10 sec stimulation - 9 minutes wait - 10 sec stimulation - 3 minutes wait - 45 seconds of final rinse with sterilized and ozonated water.
- Removing the slime collection bottle, performing a washing of 4 min of the animal with sterilized and ozonated water before going back in the culture environments.

### Example 3:

- First rinsing to eliminate the coarse impurities (earth, little stones and grass).
- Transferring 10 kg of snails (700/720 snails) in the first container for washing with water and ozone.
- In the first container, washing for a duration of 30 min with sterilized and ozonated water.
- Transferring the washed snails in the basket within the second container for the extraction.
- Extraction: 30 sec stimulation - 12 min wait - 10 sec stimulation - 12 min wait - 10 sec stimulation - 12 minutes wait - 10 sec stimulation - 5 minutes wait - 45 seconds of final rinse with sterilized and ozonated water.
- Removing the slime collection bottle, performing a washing of 4 min of the animal with sterilized and ozonated water before going back in the culture environments.

### Example 4:

- First rinsing to eliminate the coarse impurities (earth, little stones and grass).
- Transferring 10 kg of snails (700/720 snails) in the first container for washing with water and ozone.
- In the first container, washing for a duration of 30 min with sterilized and ozonated water.
- Transferring the washed snails in the basket within the second container for the extraction.
- Extraction: 40 sec stimulation - 12 min wait - 10 sec stimulation - 12 min wait - 10 sec stimulation - 12 minutes wait - 10 sec stimulation - 5 minutes wait - 90 seconds of final rinse with ozonated and sterilized water.
- Removing the slime collection bottle, performing a washing of 4 min of the animal with sterilized and ozonated water before going back in the culture environments.

The different examples result from the study of the method to obtain different concentrations of extract.

A preferred embodiment for the stimulating solution is as follows (table 1):

**Table 1**

| Ingredient | Amount in weight % |
|---|---|
| Citric acid (granulated) monohydrate | 5 |
| Sodium benzoate | 0.1 |
| Potassium sorbate | 0.05 |
| N,N,N-trimethylglycine* | 1 % |
| xylitol | 1 % |
| purified water | 92.85 % |

| | |
|---|---|
| *e.g. Genencare OSMS BH (DuPont) | |

Implementing the invention, the use of the device for the extraction of snail slime, the device, the stimulating solution and its use and the extraction process of the invention may be modified according to executive modifications and variants not described herein. If such modifications or such variants should fall within the scope of the following claims, they should all be considered protected by the present patent.

## Claims

1. Use of a device (10) comprising:
(a) at least one container (14, 16) with at least one hole (38, 28) and its lid (20, 22) to close the container (14, 16) hermetically, where the container (14, 16) or the lid (20, 22) is provided with at least an inlet (18, 24, 26) in order to introduce a stimulating solution or sterilized and/or ozonated water;
(b) a sanitizing unit comprising an ozone generator (32) and a sterilization device (36), and which can be supplied or is supplied by a water supply;
(c) one or more tanks (40) for containing or that contain a stimulating solution; and
(d) a liquid transport system, for the transfer of the stimulating solution and the sterilized and/or ozonated water respectively from said one or more tanks (40) and from said sanitizing unit through said inlet (18, 24, 26) to said at least one container (14, 16);
wherein said at least one inlet (18, 24, 26) is connectable or is connected with said one or more tanks (40) or said sanitizing unit in order to be supplied with said stimulating solution or said sterilized and/or ozonated water
for the extraction of snail slime.

2. Use according to claim 1, **characterized in that** said device (10) includes:
(a) at least a first (14) and a second (16) container, each having at least one hole (38, 28) and its lid (20, 22) to close the container hermetically, where each container (14, 16) or its lid (20, 22) is provided with at least an inlet (18, 24, 26) in order to introduce a stimulating solution or sterilized and/or ozonated water;
(b) a sanitizing unit comprising an ozone generator (32) and a sterilization device (36), and which can be supplied or is supplied by a water supply;
(c) one or more tanks (40) for containing or that contain a stimulating solution; and
(d) a liquid transport system, for the transfer of the stimulating solution from said one or more tanks (40) through the respective inlet (24) to said second container (16), and for the transfer of the sterilized and/or ozonated water from said sanitizing unit, through the respective inlet (18, 26), to said first container (14) or said second (16) container;
wherein said at least one inlet (18) of the first container (14) is connectable or is connected with said sanitizing unit to be supplied with sterilized and/or ozonated water, and wherein said at least one inlet (24, 26) of the second container is connectable or is connected with said one or more tanks (40) or with said sanitizing unit to be supplied with said stimulating solution or said sterilized and/or ozonated water.

3. Use according to claim 2, **characterized in that** said second container (16) or its lid (22) comprises a first (26) and a second (24) inlet, where the first inlet (26) is connectable or is connected with said sanitizing unit and where said second inlet (24) is connectable or is connected with said one or more tanks (40) for containing or that contain said stimulating solution.

4. Use according to any one of the preceding claims, **characterized in that** said inlet(s) (18, 24, 26) of said container(s) (14, 16) is/are provided with a spray device.

5. Use according to any one of the preceding claims, **characterized in that** the following steps are performed:
(a) treatment of snails, preferably of the species *Helix aspersa Müller,* with ozonated water; and
(b) treatment of the snails coming from step (a) with a stimulating solution having a pH between 1.4 and 2.6 and comprising, as an aqueous solution, citric acid, sodium benzoate and potassium sorbate and, optionally, betaine N,N,N-trimethylglycine and xylitol.

6. Use according to claim 5, **characterized in that** the solution comprises from 4 to 15% by weight, preferably from 4 to 8% by weight, more preferably about 5% by weight of citric acid, from 0.05 to 0.9% by weight, preferably from 0.05 to 0.5% by weight, more preferably 0.1 % by weight of sodium benzoate, and from 0.03 to 0.9% by weight, preferably from 0.03 to 0.5% by weight, more preferably about 0.05% by weight of potassium sorbate, and **by the fact** that - in case of their presence - the quantities of betaine and xylitol each vary between 0.5 and 2% by weight, and correspond preferably to about 1% by weight.

7. Use according to one of claims 5 or 6, **characterized in that** in said step (b) alternate treatments with stimulating solution and standby steps without treatment of the snails are used.

8. Stimulating solution for the stimulation of production of snail slime having a pH between 1.4 and 2.6 and comprising, as an aqueous solution, citric acid, sodium benzoate and potassium sorbate and, optionally, betaine N,N,N-trimethylglycine and xylitol.

9. Solution for the stimulation of production of snail slime according to claim 8, **characterized in that** the solution comprises from 4 to 15% by weight, preferably from 4 to 8% by weight, more preferably about 5% by weight of citric acid, from 0.05 to 0.9% by weight, preferably from 0.05 to 0.5% by weight, more preferably 0.1 % by weight of sodium benzoate, and from 0.03 to 0.9% by weight, preferably from 0.03 to 0.5% by weight, more preferably about 0.05% by weight of potassium sorbate, and **by the fact** that - in case of their presence - the quantities of betaine and xylitol each vary between 0.5 and 2% by weight, and correspond preferably to about 1 % by weight.

10. Use of the solution according to claim 8 or 9 for stimulation of the production of snail slime for the extraction of snail slime.

11. Process for the extraction of snail slime comprising the following steps:
(a) treatment of snails, preferably of the species *Helix aspersa Müller,* with ozonated water; and
(b) treatment of snails from step (a) with a stimulating solution according to claim 8 or 9.

12. Process according to claim 11, **characterized in that** in said step (b) alternate treatments with stimulating solution and standby steps without treatment of the snails are used.

13. Device for the extraction of snail slime comprising
(a) at least a first (14) and a second (16) container, each having at least one hole (38, 28) and its lid (20, 22) to close the container hermetically, where each container (14, 16) or its lid (20, 22) is provided with at least an inlet (18, 24, 26) in order to introduce a stimulating solution or sterilized and/or ozonated water;
(b) a sanitizing unit comprising an ozone generator (32) and a sterilization device (36), preferably a UV lamp or a reverse osmosis device, and which can be supplied or is supplied by a water supply;
(c) one or more tanks (40) for containing or that contain a stimulating solution; and
(d) a liquid transport system, for the transfer of the stimulating solution from said one or more tanks (40) through the respective inlet (24) to said second container (16), and for the transfer of the sterilized and/or ozonated water from said sanitizing unit, through the respective inlet (18, 26), to said first container (14) or said second (16) container;
wherein said at least one inlet (18) of the first container (14) is connectable or is connected with said sanitizing unit to be supplied with sterilized and/or ozonated water, and wherein said at least one inlet (24, 26) of the second container is connectable or is connected with said one or more tanks (40) or with said sanitizing unit to be supplied with said stimulating solution or said sterilized and/or ozonated water.

14. Device according to claim 13, **characterized in that** said second container (16) or its lid (22) comprises a first (26) and a second (24) inlet, where the first inlet (26) is connectable or is connected with said sanitizing unit and where said second inlet (24) is connectable or is connected with said one or more tanks (40) for containing or that contain said stimulating solution.

15. Device according to any one of claims 13 to 14, **characterized in that** it comprises, in at least one of said tanks, a solution for the stimulation of production of snail slime, in particular a stimulating solution having a pH between 1.4 and 2,6 and comprising, as an aqueous solution, citric acid, sodium benzoate and potassium sorbate and, optionally, betaine N,NN-trimethylglycine and xylitol, and wherein the solution preferably comprises from 4 to 15% by weight, more preferably from 4 to 8% by weight, still more preferably about 5% by weight of citric acid, preferably from 0.05 to 0.9% by weight, more preferably from 0.05 to 0.5% by weight, still more preferably 0.1% by weight of sodium benzoate, and preferably from 0.03 to 0.9% by weight, more preferably from 0.03 to 0.5% by weight, still more preferably about 0.05% by weight of sorbate potassium, and by the fact that - in case of their presence - the quantities of betaine and xylitol each vary between 0.5 and 2% by weight, and preferably correspond to about 1% by weight.
